# EUROPEAN PATENT APPLICATION

(11) **EP 4 497 394 A1**
(43) Date of publication of application: **29.01.2025**
(21) Application number: 22935799.1
(22) Date of filing: 29.03.2022
(51) Int. Cl.: A61B 10/02, A61B 17/34, A61B 34/30

(54) **ROBOTIC APPARATUS USING MEDICAL IMAGING EQUIPMENT FOR CONTROLLING BIOPSY NEEDLE FOR REAL-TIME BIOPSIES**

(30) Priority: 28.03.2022 KR 20220038233
(71) Applicant: Cure-in Inc., Goyang-si, Gyeonggi-do 10407 (KR); National Cancer Center, Goyang-si, Gyeonggi-do 10408 (KR)
(72) Inventor: JO, Yung Ho, Goyang-si Gyeonggi-do 10222 (KR); LEE, Sun Ok, Paju-si Gyeonggi-do 10893 (KR); PARK, Byoeng Jun, Goyang-si Gyeonggi-do 10417 (KR); KANG, Han Sung, Goyang-si Gyeonggi-do 10416 (KR); YOON, Chae Hyun, Ansan-si Gyeonggi-do 15399 (KR)
(74) Representative: RGTH
(86) International application number: PCT/KR2022/004396
(87) International publication number: WO 2023/191123

(57) **Abstract**

Disclosed is a robotic apparatus for controlling a biopsy needle, the robotic apparatus controlling a biopsy needle module to insert a needle part thereof into a biopsy area of a patient on a bed equipped with medical imaging equipment, and biopsy a tissue sample, and the robotic apparatus comprising: a biopsy needle module (100) in which a needle part (110) is provided with overlapping outer needle (111) and inner needle (113), and an inner needle-fixing rib (114) fixed and coupled to the rear end of the inner needle (113) and an outer needle-fixing rib (112) provided in front of the inner needle-fixing rib (114) and coupled to the rear end of the outer needle (111); an end-effector (300), which is provided on a side of the bed (11) and into which the inner needle-fixing rib (114) and outer needle-fixing rib (112) are fitted and coupled, for moving the inner needle-fixing rib (114) and outer needle-fixing rib (112) forward and backward for the biopsy after the needle part (110) has penetrated the target location in the biopsy area of a patient; and a location-controlling robot (400), provided behind the end-effector (300) by a set distance, for controlling the horizontal and vertical directions and insertion depth of the end-effector (300) relative to the bed so that the needle part (110) penetrates the target location in the biopsy area.

## Description

### [Technical Field]

The present invention relates to a biopsy needle manipulation robotic apparatus, and more particularly to a biopsy needle manipulation robotic apparatus that manipulates a biopsy needle module to accurately insert a biopsy needle into a biopsy site of a patient while checking an image of the biopsy site of the patient in real time using medical imaging equipment.

### [Background Art]

A biopsy is a type of examination method in which a hollow needle is inserted into an organ in vivo without making an incision in the skin to collect a part of the tissue for pathological histological examination. A biopsy is a preoperative examination of a suspected tumor by sonography, computed tomography (CT), magnetic resonance imaging (MRI), etc. in the initial diagnosis of cancer, and a biopsy is taken based on images.

When taking a biopsy using medical images such as CT or MR, the space in a gantry of medical imaging equipment where a patient is located is usually narrow, and a general biopsy needle is straight and long, taking up a lot of space, whereby it is difficult to introduce the needle into the patient's tissue in the small gantry of the medical imaging equipment.

In order to solve this problem, a biopsy robot system that performs a biopsy procedure on a patient while checking an image in real time in a gantry using a bendable needle device is disclosed in Korean patent Application Publication No. 10-2016-0127653.

In the disclosed "bendable needle device and real-time biopsy robotic system using the same," a robotic end effector moves the bendable needle device to the side of a bed where a patient is lying on his or her stomach using a robot controller such that an operator can insert a needle device into a biopsy site and biopsy a tissue sample in the gantry while checking an image.

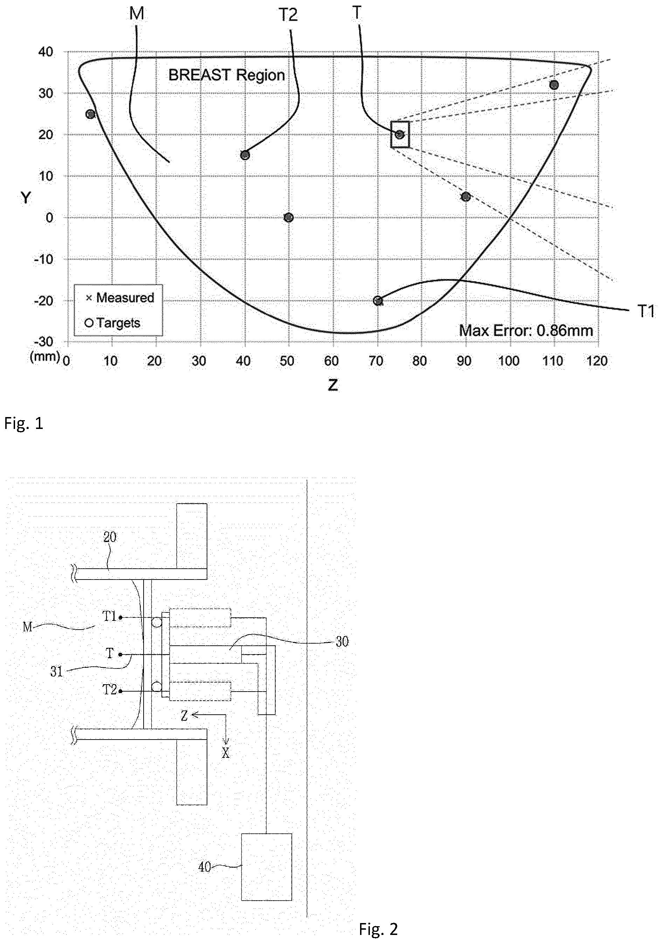

FIG. 1 shows an example of a breast image captured by medical imaging equipment. As shown, tissue samples are taken from the breast, which is a biopsy site M, at various target positions T, T1, and T2.

In the disclosed conventional biopsy robot system, a fixing frame 20 configured to fix a region to be biopsied is disposed at the side of the breast with the patient prone, as shown in FIG. 2. A needle 31 of a bendable needle device 30 is then inserted into the biopsy site using a robot end effector 40.

However, the conventional biopsy robot system has a limitation that it is difficult to accurately insert the needle 31 of the bendable needle device 30 into various target positions because the operator performs manipulation from a distance using a navigation unit. That is, it is difficult to accurately adjust the bendable needle device 30 relative to a bed 11 in a horizontal direction and a vertical direction.

### [Disclosure]

### [Technical Problem]

The present invention has been made in view of the above problems, and it is an object of the present invention to provide a biopsy needle manipulation robotic apparatus capable of manipulating the position of a biopsy needle module such that the biopsy needle module can be accurately inserted into a biopsy target position in a biopsy site to biopsy a tissue sample.

It is another object of the present invention to provide a biopsy needle manipulation robotic apparatus that can be used compatibly with various types of medical imaging equipment.

The above objects and various advantages of the present invention will become apparent to those skilled in the art from a preferred embodiment of the present invention.

### [Technical Solution]

The above objects of the present invention may be accomplished by a biopsy needle manipulation robotic apparatus for manipulating a biopsy needle module such that a needle unit of the biopsy needle module is inserted into a biopsy site of a patient lying on a bed for medical imaging equipment to biopsy a tissue sample. The needle unit (110) of the biopsy needle module (100) includes an outer needle (111) and an inner needle (113) overlapping each other, an inner needle fixing rib (114) fixedly coupled to a rear end of the inner needle (113), and an outer needle fixing rib (112) provided in front of the inner needle fixing rib (114), the outer needle fixing rib being fixedly coupled to a rear end of the outer needle (111), and the biopsy needle manipulation robotic apparatus according to the present invention includes an end effector (300) provided on a side surface of the bed (11), the end effector being configured to allow the inner needle fixing rib (114) and the outer needle fixing rib (112) to be coupled thereto by fitting, the end effector being configured to move each of the inner needle fixing rib (114) and the outer needle fixing rib (112) forward and backward such that the needle unit (110) penetrates the biopsy site of the patient to perform a biopsy procedure, and a position adjustment robot (400) provided at the rear of the end effector (300) so as to have a predetermined length, the position adjustment robot being configured to adjust the horizontal position, the vertical position, and the insertion depth of the end effector (300) with respect to the bed such that the needle unit (110) penetrates a target position of the biopsy site.

In an embodiment, the end effector (300) may include a casing unit (310) disposed vertically on a side surface of the bed (11), a trigger module (320) coupled to the interior of the casing unit (310) so as to be movable leftward and rightward, the trigger module being configured to allow the outer needle fixing rib (112) and the inner needle fixing rib (114) of the biopsy needle module (100) to be coupled thereto, the trigger module being configured to move the biopsy needle module (100), a module transfer driving unit (330) including a module transfer shaft (333) extending through the trigger module (320) and horizontally provided at a lower part of the casing unit (310) and a module transfer motor (331) configured to forwardly and reversely rotate the module transfer shaft (333), the module transfer driving unit (330) being configured to forwardly or backwardly move the trigger module (320) toward or away from the biopsy site by forward and reverse rotation of the module transfer shaft (333) and to adjust the position of the inner needle (113), and an outer needle driving shaft (343) extending through the trigger module (320) and horizontally disposed at an upper part of the module transfer shaft (333) so as to be coupled to the outer needle fixing rib (112) and an outer needle driving motor (341) configured to forwardly and reversely rotate the outer needle driving shaft (343).

In an embodiment, the trigger module (320) may be provided at an upper part thereof with a carrier movement path (321-2) having a predetermined length, the trigger module may include an outer needle carrier (325) having an outer needle rib insertion recess (325a), into which the outer needle fixing rib

(112) is inserted, formed on an upper surface thereof, the outer needle carrier being integrally provided at a lower part thereof with a moving block (325-2) coupled so as to be movable forward and backward along the carrier movement path (321-2), the moving block having a handle movement rail (325-4) recessed inward in both side surfaces of a lower part thereof, a handle (326) coupled on a path of the outer needle driving shaft (343), the handle having a thread formed at an outer circumferential surface thereof, the handle being provided at one side thereof with a carrier pressing rib (326-1) configured to move along the handle movement rail (325-4) and to press the outer needle carrier (325) such that the outer needle carrier (325) is moved forward and backward, the a carrier pressing rib protruding from the one side of the handle, a hinge (328) coupled to a lower part of the trigger module (320), the hinge being configured to restrict the front position and the rear position of the outer needle carrier (325) when the outer needle carrier (325) is moved forward and backward by the handle (326), and an elastic member support shaft (327) extending and disposed through the trigger module (320) and an elastic member (327a) wound around an outer circumferential surface of the elastic member support shaft (327), the elastic member being compressed upon backward movement of the trigger module (320) to provide an elastic force to allow the trigger module (320) to be moved forward, a front hinge protrusion (325-5) may be formed on the front of the handle movement rail (325-4) so as to protrude outward therefrom, and a movement restriction stopper (325-6) is formed on the rear of the handle movement rail so as to protrude downward therefrom, side protrusions (328-3) may be formed on both sides of a rear end of the hinge (328) so as to protrude upward therefrom, and a rib insertion slope (328-4) may be formed between the pair of side protrusions (328-3) so as to be inclined rightward and upward, when the outer needle carrier (325) is backwardly moved by the handle (326), the front hinge protrusion (325-5) may be moved along the side protrusions (328-3) and then caught by the side protrusions (328-3), whereby forward movement of the front hinge protrusion may be restricted, and a front end of the carrier pressing rib (326-1) of the handle (326) may be formed in a shape corresponding to the rib insertion slope (328-4) such that the handle (326) is moved forward by driving of the outer needle driving shaft (343), the carrier pressing rib (326-1) may be inserted into the rib insertion slope (328- 4), and the carrier pressing rib (326-1) may be pressed to release the state in which the front hinge protrusion (325-5) is caught by the side protrusions (328-3).

In an embodiment, the casing unit may be provided at a lower part and an upper part of the rear thereof with a lower arm pivot axis (313b) and an upper arm pivot axis (313a), respectively, the position adjustment robot (400) may include a robot casing (410) disposed vertically at the rear of the casing unit (310), a lower arm driving unit (440) having a lower arm moving nut (455) disposed horizontally at a lower part of the robot casing (410), an upper arm driving unit (450) having an upper arm moving nut (455) disposed horizontally on an upper part of the robotic casing (410), a lower sliding arm (420) configured to horizontally connect the lower arm moving nut (455) to a rear end of the lower part of the casing unit (310), an upper sliding arm (430) configured to horizontally connect the upper arm moving nut (455) to a rear end of an upper part of the casing unit (310), and a lower arm pivot axis connector (460) and an upper arm pivot axis connector (470) configured to connect ends of the lower sliding arm (420) and the upper sliding arm (430) to the lower arm pivot axis (313b) and the upper arm pivot axis (313a), respectively, a change in length of each of a lower arm driving shaft (443) and an upper arm driving shaft (453) may be converted into a change in length of at least one of the lower sliding arm (420) and the upper sliding arm (430), and the change in length of at least one of the lower sliding arm (420) and the upper sliding arm (430) may be converted into upward and downward tilting of the casing unit (310) about the lower arm pivot axis (313b) and the upper arm pivot axis (313a).

In an embodiment, the biopsy needle manipulation robotic apparatus may further include a position adjustment fixing jig 500 coupled to the bed (11) above the head of the patient, the position adjustment fixing jig being configured to adjust the horizontal position and the vertical height at which the position adjustment robot (400) is coupled to the bed (11).

### [Advantageous Effects]

The biopsy needle manipulation robotic apparatus according to the present invention accurately guides the needle unit of the biopsy needle module coupled to the end effector to the target position of the biopsy site of the patient.

In particular, the module transfer driving unit and the outer needle driving unit of the end effector drive the inner needle and the outer needle simultaneously or independently to accurately control the introduction depth of the needle unit and to control the biopsy to be completed accurately.

In addition, the biopsy needle manipulation robotic apparatus accurately guides the needle unit to the target position guided by the operator in the horizontal direction, the height direction, and the depth direction by manipulating the position adjustment robot through the navigation unit, enabling a precise biopsy procedure.

In addition, the present invention has the advantage of being compatible with various types of medical imaging equipment by changing the height and the coupling width using the height adjustment fixing jig.

### [Description of Drawings]

FIG. 1 is an illustrative view showing target positions of a biopsy site;
FIG. 2 is a sectional illustrative view showing a conventional state in which a bendable needle device is inserted into a biopsy site of a patient;
FIG. 3 is an illustrative view showing a state in which a biopsy procedure is performed using a biopsy system to which a biopsy needle manipulation robotic apparatus according to the present invention is applied;
FIG. 4 is a perspective view showing a biopsy system according to the present invention;
FIG. 5 is a view showing a process in which a biopsy needle module is coupled to the biopsy needle manipulation robotic apparatus according to the present invention;
FIG. 6 is a perspective view showing the configuration of the biopsy needle module coupled to the biopsy needle manipulation robotic apparatus according to the present invention;
FIG. 7 is an illustrative view showing a state in which the biopsy needle module is coupled to an end effector;
FIG. 8 is an exploded perspective view showing the configuration of the end effector;
FIG. 9 is an enlarged view showing a coupling structure of a trigger module of the end effector;
FIGs. 10 and 11 are illustrative views showing processes in which the biopsy needle module is operated by the end effector;
FIG. 12 is a plan view showing the operation of the end effector in each step;
FIG. 13 is a bottom view showing the position of a handle of the end effector in each step;
FIG. 14 is a sectional view showing a change in position of an outer needle carrier, the handle, and a hinge member of the end effector in each step;
FIG. 15 is an exploded perspective view showing a coupling structure of a position adjustment robot and a position adjustment fixing jig;
FIG. 16 is a perspective view showing a state in which the position adjustment robot is coupled to the position adjustment fixing jig;
FIG. 17 is an internal perspective view showing an internal configuration of the position adjustment robot;
FIG. 18 is a view showing a coupling structure of the position adjustment robot and a pivot axis of the end effector;
FIG. 19 is a plan sectional view showing the coupling structure of the position adjustment robot and the pivot axis of the end effector; and
FIG. 20 is an illustrative view showing a state in which the end effector is tilted by the position adjustment robot.

### [Best Mode]

In order to fully understand the present invention, a preferred embodiment of the present invention will be described with reference to the accompanying drawings. The embodiment of the present invention may be changed in various forms, and the scope of the present invention must not be interpreted as being limited to the following embodiment described below in detail. The present embodiment is provided to more completely describe the present invention to a person having ordinary skill in the art to which the present invention pertains. Consequently, the shapes, etc. of elements in the drawings may be exaggerated for clearer description. It should be noted that identical members may be denoted by the same reference numerals in the drawings. A detailed description of related known functions and constructions will be omitted when the same may obscure the subject matter of the present invention.

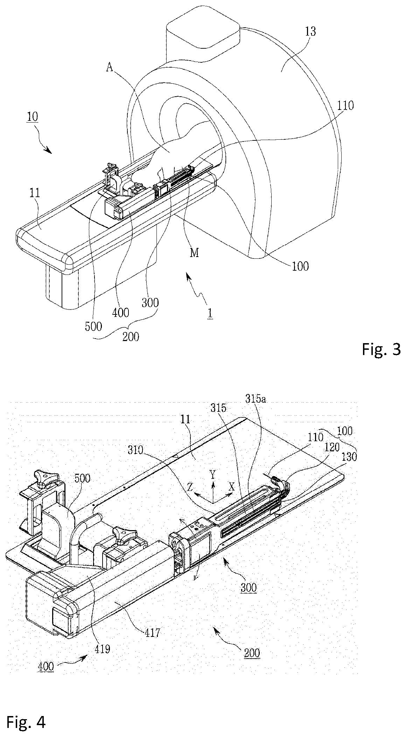

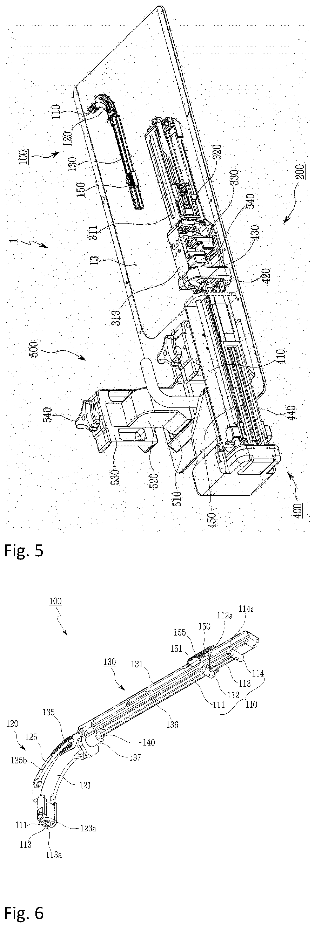

FIG. 3 is an illustrative view showing a biopsy system 1 that performs a biopsy procedure through medical imaging equipment 10 using a biopsy needle manipulation robotic apparatus 200 according to the present invention, FIG. 4 is a perspective view showing the configuration of the biopsy system 1, and FIG. 5 is an exploded perspective view showing a coupling structure of the biopsy needle manipulation robotic apparatus 200 and a biopsy needle module 100.

As shown in FIG. 3, the biopsy needle manipulation robotic apparatus 200 according to the present invention is applied to a biopsy system 1 and is used to adjust the position of a biopsy needle module 100 in a real-time biopsy procedure using medical imaging equipment. The biopsy system 1 is coupled to a bed 11 of medical imaging equipment, such as an MRI, such that an operator can accurately biopsy a biopsy sample by inserting a needle into the position of a biopsy target while viewing an image in real time.

The biopsy system 1 includes a biopsy needle module 100 that is inserted into a biopsy site of a patient and a biopsy needle manipulation robotic apparatus 200 that adjusts the position of the biopsy needle module 100 such that the biopsy needle module is inserted into a biopsy target site. The biopsy needle manipulation robotic apparatus 200 includes an end effector 300 to which the biopsy needle module 100 is removably coupled and which manipulates the biopsy needle module 100 and a position adjustment robot 400 that adjusts the position of the end effector 300 such that the biopsy needle module 100 can be inserted into various biopsy target sites.

The biopsy needle manipulation robotic apparatus 200 is provided in a straight line at a side surface of the bed 11 on which the patient lies face down. The biopsy needle module 100 is removably coupled to the end effector 300 of the biopsy needle manipulation robotic apparatus 200 such that a bent needle unit 110 is inserted into the biopsy location of the lying patient. This allows the needle unit 110 to be inserted into the biopsy site of the patient in a confined space between the bed 11 and an inner wall surface of a gantry 13 to perform a biopsy procedure.

The operator checks an image displayed on the screen using an externally provided navigation unit (not shown) and drives the biopsy needle manipulation robotic apparatus 200 to adjust the biopsy needle module 100 to be inserted accurately into the target site.

The biopsy needle manipulation robot apparatus 200 includes an end effector 300 to which the biopsy needle module 100 is coupled and which manipulates the needle unit 110, a position adjustment robot 400 that adjusts the tilting angle of the end effector 300 in the forward-backward and upward-downward directions, and a position adjustment fixing jig 500 that adjusts the position and height of the position adjustment robot 400 at which the position adjustment robot is coupled to the bed 11.

As shown in FIG. 3, the position adjustment robot 400 is coupled to the outermost side of the bed 11 by the position adjustment fixing jig 500 to secure a space for the patient to lie on the bed. The end effector 300 is coupled to a front end of the position adjustment robot 400 so as to be tilted upward and downward, and the biopsy needle module 100 is coupled to a plate surface.

As shown in FIG. 4, the movement of the biopsy needle module 100 in an X-axis direction is performed by the position adjustment robot 400, and the movement of the biopsy needle module in a Y-axis direction is performed by tilting of the position adjustment robot 400. The movement of the biopsy needle module 100 in a Z-axis direction is realized as the invasion depth of the needle unit 110 by the end effector 300.

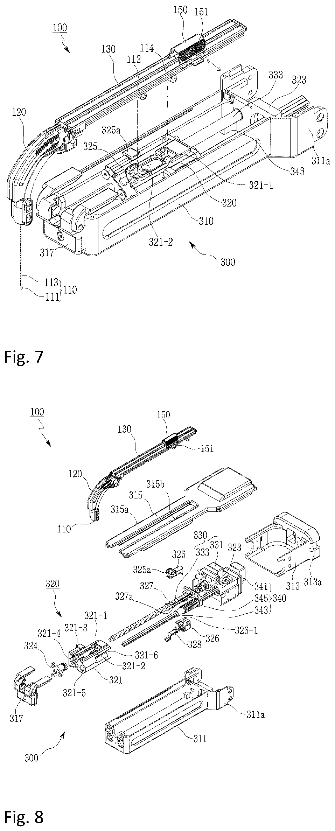

FIG. 6 is a perspective view showing the configuration of the biopsy needle module 100 removably coupled to the end effector 300 of the biopsy needle manipulation robotic apparatus 200, FIG. 7 is an illustrative view showing a process of coupling the biopsy needle module 100 to the end effector 300, and FIG. 8 is an exploded perspective view showing the configuration of the end effector 300.

The end effector 300 is coupled to the biopsy needle module 100 to manipulate the needle unit 110 of the biopsy needle module 100 such that an inner needle 113 and an outer needle 111 of the needle unit 110 move toward or away from a biopsy site M together or separately.

Here, as shown in FIG. 6, the biopsy needle module 100 includes a needle unit 110, a needle bending frame 120 coupled to the needle unit 110 with a front end of the needle unit 110 inserted therein, the needle bending frame having a bent shape, and a needle guide 130 coupled to an upper part of the needle bending frame 120 so as to be movable along the bent shape of the needle bending frame 120, the needle guide being configured to support the needle unit 110.

The needle unit 110 is manipulated by the end effector 300 and is inserted into a biopsy target site T to biopsy a tissue sample S. The needle unit 110 is configured such that the inner needle 113 and the outer needle 111 overlap each other. The inner needle 113 is formed with a larger length than the outer needle 111.

An outer needle fixing rib 112 coupled to the end effector 300 is fixedly coupled to the rear of the outer needle 111, and a front guide coupling protrusion (not shown) coupled to the needle guide 130 is formed on an upper part of the outer needle fixing rib 112 so as to protrude therefrom.

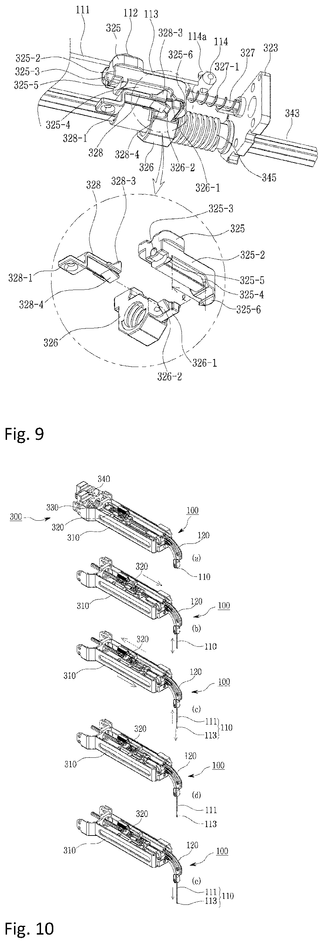

A sharp tip 113a is provided at the front end of the inner needle 113, and a biopsy recess 113b (see FIG. 10) depressed from the plate surface to biopsy the tissue sample is formed at the rear of the tip 113a. The rear of the inner needle 113 is exposed to the outer side of the outer needle 111 by a predetermined length and is coupled to an inner needle fixing rib 114. The inner needle fixing rib 114 is coupled to the end effector 300, and a rear guide coupling protrusion (not shown) is formed on an upper part of the inner needle fixing rib 114 so as to protrude therefrom.

As shown in FIG. 8, the end effector 300 includes a casing unit 310 disposed vertically on the side surface of the bed 11, a trigger module 320 which is coupled to the interior of the casing unit 310 so as to be movable in a horizontal direction (X-axis direction), into which the outer needle fixing rib 112 and the inner needle fixing rib 114 of the biopsy needle module 100 are inserted, and which moves the biopsy needle module 100, a module transfer driving unit 330 horizontally provided under the casing unit 310 through the trigger module 320, the module transfer driving unit being configured to move forward and backward through the trigger module 320 and to forwardly or backwardly move the inner needle 113 and the outer needle 111 toward or away from the biopsy site, and an outer needle driving unit 340 provided in parallel with the module transfer driving unit 330, the outer needle driving unit being configured to move the outer needle 111 forward or backward separately from the inner needle 113.

The casing unit 310 is disposed at a side surface of an upper part of the bed 11 in a vertical direction and movably supports the biopsy needle module 100 after the biopsy needle module is coupled to an externally exposed biopsy needle coupling recess 315a. The casing unit 310 includes a casing body 311, a robot coupling cap 313 coupled to a rear end of the casing body 311, the robot coupling cap being configured to connect the casing body 311 and the position adjustment robot 400 to each other, a cover 315 configured to cover upper surfaces of the casing body 311 and the robot coupling cap 313, and a front cap 317 coupled to the front of the casing body 311.

The trigger module 320, a module transfer shaft 333 of the module transfer driving unit 330, and an outer needle driving shaft 343 of the outer needle driving unit 340 are received in the casing body 311. As shown in FIG. 5, the module transfer shaft 333 and the outer needle driving shaft 343 are located side by side above and below in the casing body 311, and the trigger module 320 is coupled to be moved in a leftward-rightward direction through the module transfer shaft 333 and the outer needle driving shaft 343.

A pair of cap coupling wings 311a is formed on both sides of the rear of the casing body 311 so as to extend therefrom and is coupled to the robot coupling cap 313. A rear wall 311b is formed at the boundary of the casing body 311 and the cap coupling wing 311a to separate a space in the casing body 311 from a space in the robot coupling cap 313.

The robot coupling cap 313 is coupled to the rear end of the casing body 311 and is connected to the position adjustment robot 400. A module transfer motor 331 of the module transfer driving unit 330 and an outer needle driving motor 341 of the outer needle driving unit 340 are received in the robot coupling cap 313.

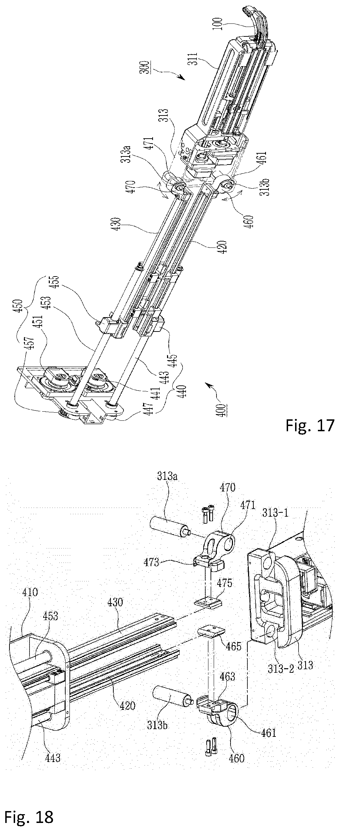

An upper arm pivot axis 313a and a lower arm pivot axis 313b are provided at both sides of the rear of the robot coupling cap 313, respectively. As shown in FIG. 18, the upper arm pivot axis 313a is coupled to an upper arm pivot axis connector 470 of the position adjustment robot 400, and the lower arm pivot axis 313b is coupled to a lower arm pivot axis connector 460 to accommodate a change in the length of an upper sliding arm 430 and a lower sliding arm 420. As a result, the casing unit 310 is tilted in the upward-downward direction to adjust the height at which the needle unit 110 of the biopsy needle module 100 is inserted into the biopsy site M.

The front cap 317 is coupled to the front of the casing body 311. The needle bending frame 120 of the biopsy needle module 100 is seated in the front cap 317 to support the needle unit 110 such that the needle unit can be bent and inserted into the biopsy site M.

The cover 315 covers upper parts of the casing body 311 and the robot coupling cap 313. The cover 315 hides the module transfer shaft 333, the outer needle driving shaft 343, and the trigger module 320 received in the casing unit 310 and allows only the biopsy needle module 100 to be mounted to the trigger module 320. To this end, the biopsy needle coupling recess 315a is formed in the cover 315 by cutting, and the biopsy needle module 100 is coupled through the biopsy needle coupling recess 315a.

As shown in FIG. 7, the operator inserts the inner needle fixing rib 114 and the outer needle fixing rib 112 respectively into an inner needle rib insertion recess 321-1 and an outer needle rib insertion recess 325a of the trigger module 320 exposed to the outside through the biopsy needle coupling recess 315a, presses a locking member 150, and inserts a locking button 151 into a button coupling recess 315b formed in an inner wall surface of the biopsy needle coupling recess 315a to complete coupling between the biopsy needle module 100 and the end effector 300.

The trigger module 320 is coupled to the outer needle fixing rib 112 and the inner needle fixing rib 114 of the biopsy needle module 100 to support the outer needle 111 and the inner needle 113 so as to be moved forward and backward by the driving force of the module transfer driving unit 330 and the outer needle driving unit 340.

As shown in FIG. 8, the trigger module 320 includes a module body 321, a rear block 323 coupled to the rear of the module body 321, a front block 324 coupled to the front of the module body 321, an outer needle carrier 325 movably coupled to an upper part of the module body 321 and coupled to the outer needle fixing rib 112, a handle 326 coupled to the outer needle driving shaft 343 of the outer needle driving unit 340 and configured to move the outer needle carrier 325 backward, a hinge 328 coupled to a lower part of the trigger module 320 and configured to fix the position of the outer needle carrier 325 backwardly moved by the handle 326, and an elastic member 327a coupled to the trigger module 320 and configured to forwardly move the trigger module 320 backwardly moved by the hinge 328 such that the outer needle 111 biopsies the tissue sample S.

The module body 321 receives the outer needle fixing rib 112 and the inner needle fixing rib 114 of the biopsy needle module 100 on an upper surface thereof, and is coupled to the module transfer shaft 333 and the outer needle driving shaft 343 to move the casing unit 310 forward and backward in conjunction with the forward and reverse rotation of the module transfer shaft 333. The needle unit 110 is also moved forward and backward in conjunction with the forward and backward movement of the module body 321, and is inserted into or backwardly moved from the biopsy site M.

The inner needle rib insertion recess 321-1, into which the inner needle fixing rib 114 is inserted, is formed in the upper surface of the module body 321. In front of the inner needle rib insertion recess 321-1, a carrier movement path 321-2 is formed by a certain length. The outer needle carrier 325, into which the outer needle fixing rib 112 is inserted, is coupled to the carrier movement path 321-2.

An elastic shaft insertion hole 321-3 to which an elastic member support shaft 327, around which the elastic member 327a is wound, is coupled is formed in the front of the module body 321. In addition, a module transfer shaft insertion hole 321-4, through which the module transfer shaft 333 is inserted on both sides, and an outer needle driving shaft insertion hole 321-5, through which the outer needle driving shaft 343 is inserted, are formed in a lower part of the module body 321. A thread corresponding to the module transfer shaft 333 is formed in the module transfer shaft insertion hole 321-4, whereby the module body 321 moves forward and backward along the module transfer shaft 333 in conjunction with the forward and reverse rotation of the module transfer shaft 333.

In addition, a screw tube coupling recess 321-6 that exposes the outer needle driving shaft 343 to the outside is provided at the rear of the outer needle driving shaft insertion hole 321-5 of the module body 321. A handle transfer screw tube 345 coupled to the outer needle driving shaft 343 is coupled to the screw tube coupling recess 321-6.

The handle transfer screw tube 345 is fixedly coupled to the outer needle driving shaft 343 to transmit the forward and reverse rotation of the outer needle driving shaft 343 to the handle 326, which is threadedly coupled to the surface thereof, causing the handle 326 to move forward and backward.

The rear block 323 is coupled to the rear of the module body 321 and fixes the positions of the elastic member support shaft 327 and the handle transfer screw tube 345. When the module body 321 is moved backward, the rear block 323 comes into contact with the rear wall 311b to restrict the backward movement of the module body.

The front block 324 is coupled to the front of the module body 321 and rotatably supports the module transfer shaft 333.

The outer needle carrier 325 is coupled to the carrier movement path 321-2 of the module body 321 and moves forward and backward along the carrier movement path 321-2 in conjunction with the forward and backward movement of the handle 326. FIG. 9 is an enlarged view showing the coupling configuration around the outer needle carrier 325, excluding the module body 321 and an enlarged and exploded perspective view showing the outer needle carrier 325, the hinge 328, and the handle 326.

The outer needle rib insertion recess 325a, into and to which the outer needle fixing rib 112 of the biopsy needle module 100 is inserted and fixed, is formed in the upper surface of the outer needle carrier 325. A moving block 325-2 that is moved along the carrier movement path 321-2 is integrally provided at a lower part of the outer needle carrier 325. A carrier elastic shaft insertion hole 325-3, into which the elastic member support shaft 327 is inserted, is formed through the interior of the moving block 325-2 in a longitudinal direction.

The moving block 325-2 is provided at a side surface thereof with a handle movement rail 325-4 recessed inward in the longitudinal direction. A front hinge protrusion 325-5 is formed on the front of the handle movement rail 325-4 so as to protrude therefrom, and a movement restriction stopper 325-6 is formed on the rear of the handle movement rail so as to protrude therefrom.

The handle 326 is threadedly coupled to the handle transfer screw tube 345, which is fixedly coupled to an outer circumferential surface of the outer needle driving shaft 343, and is moved forward and backward by the forward and reverse rotation of the outer needle driving shaft 343 to move the outer needle carrier 325 forward and backward.

The handle 326 is provided at one side thereof with a carrier pressing rib 326-1 that protrudes toward the moving block 325-2, moves along the handle movement rail 325-4, and presses the outer needle carrier 325. The carrier pressing rib 326-1 is located so as to engage with the movement restriction stopper 325-6 of the moving block 325-2, and transmits the backward pressure of the handle 326 to the movement restriction stopper 325-6 when the handle 326 is backwardly moved by the reverse rotation of the handle transfer screw tube 345, causing the outer needle carrier 325 to move backward.

A hinge pressing end 326-2 protrudes from a leading end of the carrier pressing rib 326-1 so as to be inclined forward. When the handle 326 is moved forward by the forward rotation of the handle transfer screw tube 345, the hinge pressing end 326-2 is inserted into a rib insertion slope 328-4 of the hinge 328 to press the hinge 328 downward.

The hinge 328 is coupled to a bottom surface of the module body 321. A hinge introduction hole (not shown) that introduces the hinge 328 thereinto is formed in the bottom surface of the module body 321. A body coupling end 328-1 bent downward and coupled to the bottom surface of the module body 321, side protrusions 328-3 formed on both sides of the rear end so as to protrude upward therefrom, and a rib insertion slope 328-4 formed so as to be inclined downward between the pair of side protrusions 328-3 are integrally formed at the hinge 328.

The hinge 328 is located at a lower part of the module body 321, and the pair of side protrusions 328-3 is moved along the handle movement rail 325-4. When the outer needle carrier 325 is moved forward and backward as the movement restriction stopper 325-6 is pressed by the carrier pressing rib 326-1, the side protrusions 328-3 are moved along the handle movement rail 325-4.

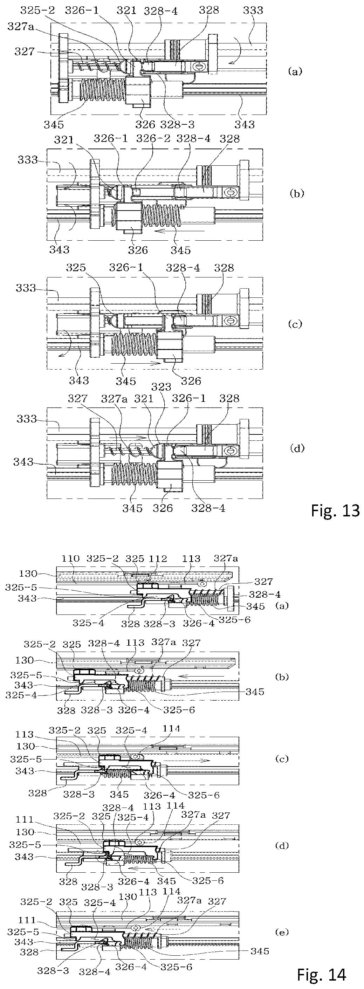

When only the outer needle carrier 325 is backwardly moved by the handle 326 in the state in which the position of the hinge 328 fixed to the module body 321 is fixed, the moving block 325-2 is moved to the rear of the side protrusions 328-3, the front hinge protrusion 325-5 is caught by the side protrusions 328-3, and the position of the outer needle carrier 325 is fixed, as shown in (c) of FIG. 14. That is, the front hinge protrusion 325-5 is caught by the pair of side protrusions 328-3, whereby the position of the outer needle carrier 325 is fixed.

The rib insertion slope 328-4 is pressed by the hinge pressing end 326-2 upon forward movement of the handle 326, whereby the state in which the front hinge protrusion 325-5 is caught by the side protrusions 328-3 is released.

As shown in (d) of FIG. 14, in the state in which the position of the outer needle carrier 325 is fixed as the result of being caught by the side protrusions 328-3, only the handle 326 is moved forward by the forward rotation of the outer needle driving shaft 343. The hinge pressing end 326-2 of the handle 326 is then inserted into the rib insertion slope 328-4. As the hinge pressing end 326-2 is inserted along the slope, the rib insertion slope 328-4 is pressed downward, and the side protrusions 328-3 integrally formed with the rib insertion slope 328-4 are moved downward.

Accordingly, the state in which the front hinge protrusion 325-5 is caught by the side protrusions 328-3 is released, whereby the outer needle carrier 325 may be shot forward, as shown in (e) of FIG. 14.

The elastic member support shaft 327 is inserted through the elastic shaft insertion hole 321-3 of the module body 321 from the rear block 323 and is supported by the front block 324. The elastic member 327a is wound around the outer circumference of the elastic member support shaft 327.

The elastic member 327a coupled to the elastic member support shaft 327 is compressed when the outer needle carrier 325 is backwardly moved in the module body 321. As shown in (a) of FIG. 13, the elastic member 327a maintains the initial length thereof when the outer needle carrier 325 is in a forwardly-moved state.

When the outer needle carrier 325 is backwardly moved by pressing of the carrier pressing rib 326-1 of the handle 326, as shown in (b) of FIG. 13, the elastic member 327a is compressed between the rear block 323 and the outer needle carrier 325.

As shown in (c) of FIG. 13, the elastic member 327a remains in a compressed state while the front hinge protrusion 325-5 is caught by the hinge 328, as shown in (d) of FIG. 13. When the state in which the front hinge protrusion 325-5 is caught by the hinge 328 is released, as shown in (d) of FIG. 13, elastic force is applied to restore the elastic member 327a to the initial length thereof, whereby the outer needle carrier 325 is rapidly moved and shot forward.

The module transfer driving unit 330 moves the trigger module 320 forward or backward. When the trigger module 320 is moved forward or backward by the module transfer driving unit 330, the inner needle 113 and the outer needle 111 coupled to the trigger module 320 are also moved forward or backward.

The module transfer driving unit 330 includes a module transfer motor 331 and a module transfer shaft 333 that is forwardly and reversely rotated by the module transfer motor 331. The module transfer motor 331 is received in the robot coupling cap 313 of the casing unit 310. The module transfer motor 331 is forwardly and reversely driven by operator manipulation through the navigation unit (not shown).

The module transfer shaft 333 is inserted into the module transfer shaft insertion hole 321-4 of the trigger module 320 through the rear wall 311b in a state of being coupled to the module transfer motor 331 and is then rotatably coupled to the front block 324. A thread corresponding to the module transfer shaft insertion hole 321-4 is formed at an outer circumferential surface of the module transfer shaft 333. Accordingly, the trigger module 320 is moved forward or backward in conjunction with the forward and reverse rotation of the module transfer shaft 333.

The outer needle driving unit 340 moves the outer needle carrier 325 coupled to the trigger module 320 forward and backward independently of the trigger module 320. The outer needle driving unit 340 includes an outer needle driving motor 341, an outer needle driving shaft 343 coupled to the outer needle driving motor 341, and a handle transfer screw tube 345 coupled to only a part of the outer needle driving shaft 343.

The outer needle driving motor 341 is received in the robot coupling cap 313 on one side of the module transfer motor 331. The outer needle driving shaft 343 is coupled to the outer needle driving motor 341, and is inserted through the outer needle driving shaft insertion hole 321-5 in the module body 321 and rotatably coupled to the front block 324.

The outer needle driving shaft 343 has no thread at an outer circumferential surface thereof and runs idle in the outer needle driving shaft insertion hole 321-5. The handle transfer screw tube 345 is coupled to the outer needle driving shaft 343 in the region corresponding to the screw tube coupling recess 321-6 of the module body 321 such that the handle 326 is moved forward and backward only by a length I of the handle movement rail 325-4.

The length I of the handle movement rail 325-4 corresponds to the distance that the outer needle carrier 325 is moved along the carrier movement path 321-2.

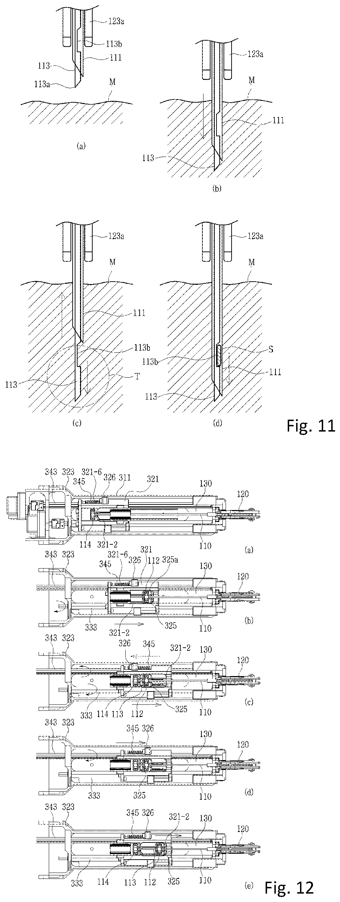

FIG. 10 is an illustrative view showing the position of the needle unit 110 of the biopsy needle module 100 for a biopsy procedure in each step, FIG. 11 is an illustrative view showing a process in which the needle unit 110 of the biopsy needle module 100 is coupled to the end effector 300 and inserted into the biopsy site M to biopsy the tissue sample S, FIG. 12 is an illustrative view showing a planar configuration of the end effector 300 in each step of the needle unit 110, FIG. 13 is an illustrative view showing a bottom configuration of the end effector 300 in each step, and FIG. 14 is an illustrative view showing a side configuration of the end effector 300 in each step.

When the biopsy needle module 100 is coupled to the end effector 300, as shown in (a) of FIG. 10, (a) of FIG. 11, and (a) of FIG. 12, the trigger module 320 is maintained in a backwardly-moved state in contact with the rear wall 311b of the casing body 311, and the needle unit 110 is located in the needle bending frame 120 in a received state.

At this time, the carrier pressing rib 326-1 of the handle 326 is maintained in contact with the movement restriction stopper 325-6 of the outer needle carrier 325, as shown in (a) of FIG. 14.

In this state, the operator moves the bed 11 into the gantry 13, manipulates the end effector 300 using the navigation unit (not shown) while viewing a captured image of the biopsy site of the patient, and manipulates the position of the needle unit 110 of the biopsy needle module 100 such that the needle unit is moved to the target biopsy site. The operator drives the module transfer motor 331 and the outer needle driving motor 341 using the navigation unit (not shown) to adjust the depth to which the inner needle 113 and the outer needle 111 penetrate the biopsy site M.

When the module transfer motor 331 is driven in the forward direction, as shown in (b) of FIG. 12, the trigger module 320 moves forward. Accordingly, the needle unit 110 moves forward, as shown in (b) of FIG. 10, and the inner needle 113 and the outer needle 111 are introduced into the biopsy site M, as shown in (b) of FIG. 11.

At this time, the carrier pressing rib 326-1 of the handle 326 moves forward in contact with the movement restriction stopper 325-6 of the outer needle carrier 325, as shown in (a) of FIG. 13 and (b) of FIG. 14.

When the needle is introduced into the biopsy site M to a predetermined depth, as shown in (b) of FIG. 11, the inner needle 113 moves forward to the target position T and the outer needle 111 moves backward to open the biopsy recess 113b of the inner needle 113, as shown in (c) of FIG. 10 and (c) of FIG. 11.

To this end, the module transfer motor 331 is rotated in the forward direction and the outer needle driving motor 341 is rotated in the reverse direction, as shown in (c) of FIG. 12. The forward rotation of the module transfer motor 331 causes the inner needle 113 and the trigger module 320 coupled to the module transfer shaft 333 to move forward. On the other hand, when the outer needle driving shaft 343 is rotated in the reverse direction by the reverse rotation of the outer needle driving motor 341, as shown in (c) of FIG. 13, the handle 326 coupled to the handle transfer screw tube 345 is moved backward.

As the handle 326 is moved backward, the carrier pressing rib 326-1 presses the movement restriction stopper 325-6 backward, whereby the outer needle carrier 325 is also moved backward. During this process, the front hinge protrusion 325-5 of the backwardly-moved outer needle carrier 325 rides over the side protrusions 328-3 of the hinge 328 and is caught by the side protrusions 328-3, whereby the position of the outer needle carrier is fixed, as shown in (c) of FIG. 14.

As the outer needle carrier 325 is moved backward, the elastic member 327a wound around the elastic member support shaft 327 remains compressed between the rear block 323 and the outer needle carrier 325.

In this state, as shown in (d) of FIG. 12, the module transfer motor 331 is stopped, and the outer needle driving motor 341 is rotated in the forward direction. As a result, the handle 326 is moved forward along the handle transfer screw tube 345.

The hinge pressing end 326-2 protruding from the carrier pressing rib 326-1 of the forwardly moved handle 326 is inserted into the rib insertion slope 328-4 of the hinge 328, as shown in (c) of FIG. 13 and (d) of FIG. 14. As the hinge pressing end 326-2 is moved inward along the rib insertion slope 328-4, the hinge pressing end presses the rib insertion slope 328-4 downward, and the side protrusions 328-3 integrally formed with the rib insertion slope 328-4 are pressed downward.

As the side protrusions 328-3 are pressed, the side protrusions are elastically moved downwardly, and the outer needle carrier 325 caught by the side protrusions 328-3 is shot forward.

As the outer needle carrier 325 is shot forward, the outer needle 111, which has been moved backward, is moved forward while covering the inner needle 113, as shown in (e) of FIG. 10 and (d) of FIG. 11.

The outer needle 111 is shot by the elastic force of the elastic member 327a and is moved forward by the length that the inner needle 113 has been moved forward, and a cutting surface formed at the front end of the outer needle 111 cuts the biopsy tissue.

In this process, the tissue sample S is received in the biopsy recess 113b and covered by the outer needle 111, whereby the biopsy procedure may be completed.

After biopsy is completed, the needle unit 110 is moved backward by the reverse driving of the module transfer motor 331 and is separated from the biopsy site of the patient.

As shown in FIGs. 4 and 5, the position adjustment robot 400 may move the end effector 300 having the biopsy needle module 100 coupled thereto forward or backward in the horizontal direction (X-axis direction), or may move the end effector upward and downward in the vertical direction (Y-axis direction), such that the needle unit 110 can be accurately inserted into the target position T of the biopsy site M.

The position adjustment robot 400 includes a robot casing 410 coupled to the position adjustment fixing jig 500 coupled to the bed 11, a lower sliding arm 420 and an upper sliding arm 430 provided at lower and upper parts of the robot casing 410, respectively, and coupled to the upper arm pivot axis 313a and the lower arm pivot axis 313b of the end effector 300, respectively, and a lower arm driving unit 440 and an upper arm driving unit 450 configured to adjust the lengths of the lower sliding arm 420 and the upper sliding arm 430, respectively.

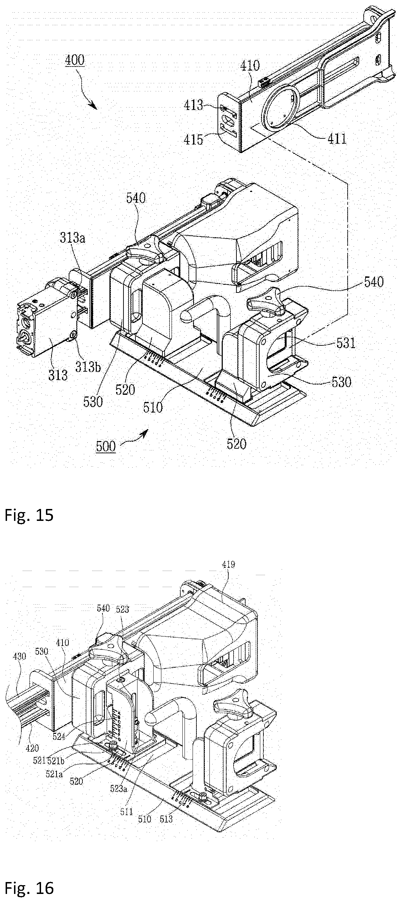

As shown in FIG. 15, the robotic casing 410 is coupled to the position adjustment fixing jig 500 to adjust the height of the biopsy needle manipulation robotic apparatus 200 coupled to the bed 11.

Here, as shown in FIG. 16, a circular jig coupling protrusion 411 is formed at the rear of the robotic casing 410 so as to protrude therefrom. The circular jig coupling protrusion 411 is fitted into and coupled to a protrusion fitting recess 531 of an adapter 530 of the position adjustment fixing jig 500.

The position adjustment fixing jig 500 includes a planar jig frame 510 fixed to the upper surface of the bed 11, a pair of position adjustment frames 520 coupled to both sides of the jig frame 510 to adjust horizontal and vertical positions at which the position adjustment robot 400 is coupled, a pair of adapters 530 coupled to the position adjustment frames 520 and coupled to the robot casing 410, and a pair of adaptor fixing handles 540 configured to constrain the coupling positions of the adapters 530 and the robot casing 410.

The position adjustment fixing jig 500 enables the biopsy needle manipulation robotic apparatus 200 to be used compatibly with various kinds of medical imaging equipment 10 having different specifications by different manufacturers.

Here, the position adjustment frames 520 and the adapters 530 are provided in pairs on both sides of the jig frame 510 to allow the biopsy manipulation robotic apparatus 200 to be coupled to the left side or the right side, depending on the orientation of the biopsy site of the patient.

The pair of jig frames 510 is disposed above the head of the patient A when the patient A is lying on the bed 11 such that the patient A does not interfere with image capture, as shown in FIG. 3.

At each side of the upper surface of the jig frame 510, a seating recess 511 is recessed from the plate surface so as to have a predetermined area. As shown in FIG. 16, the jig frame 510 is provided on a front surface thereof with a horizontal coupling position indication scale 513 indicating the position where the position adjustment frame 520 is horizontally coupled to the jig frame 510.

The position adjustment frame 520 has a predetermined height in the vertical direction and is provided at both sides of a lower part thereof with coupling flanges 521. Each coupling flange 521 is provided with a coupling width adjustment slit 521a having a predetermined length, and a coupling width adjustment screw 521b is inserted into the coupling width adjustment slit 521a.

The coupling width adjustment screw 521b extends through the coupling width adjustment slit 521a and is fixed to the jig frame 510. At this time, the operator checks the horizontal coupling position indication scale 513 and fixes the coupling width adjustment screw 521b to the position where biopsy of the patient A is facilitated.

A vertical height adjustment slit 523 is formed at the rear of the position adjustment frame 520 in a height direction, and a vertical coupling height adjustment screw 523a is inserted into the vertical height adjustment slit 523. The vertical height adjustment slit 523 is coupled through the adapters 530.

The vertical height at which the adapters 530 are coupled is adjusted by the vertical height adjustment slit 523 and the vertical coupling height adjustment screw 523a. In this case, the position adjustment frame 520 is provided with a vertical coupling height indication scale 524 to accurately adjust the height at which the adapters 530 are coupled.

The rear of the adapter 530 is coupled to the position adjustment frame 520 by the vertical coupling height adjustment screw 523a, and the front of the adapter is coupled to the robot casing 410 of the position adjustment robot 400. The adapter 530 is provided with a semicircular protrusion fitting recess 531, into and to which the circular jig coupling protrusion 411 is inserted and fixed.

The horizontal coupling position and the vertical coupling height of the position adjustment robot 400 of the biopsy needle manipulation robotic apparatus 200 are adjusted by the position adjustment fixing jig 500, whereby the biopsy system 1 of the present invention may be compatibly coupled to beds of various types of medical imaging equipment 10.

An upper arm exposure hole 413 and a lower arm exposure hole 415 are formed in the front of the robot casing 410 of the position adjustment robot 400, as shown in FIG. 15. The upper sliding arm 430 is exposed in the upper arm exposure hole 413 toward the end effector 300, and the lower sliding arm 420 is exposed in the lower arm exposure hole 415 toward the end effector 300.

The robot casing 410 is provided at the front thereof with a robot front cover 417 that covers the interior thereof, and the robot casing 410 is provided at the rear thereof with a motor cap 419 configured to receive driving motors 441 and 451 and transmission means 447 and 457 of the lower arm driving unit 440 and the upper arm driving unit 450.

The robot front cover 417 and the motor cap 419 hide driving means provided therein from the outside to reduce noise generation and dust generation when driving the medical imaging equipment, and to prevent a dangerous situation for the patient lying on the bed 11.

FIG. 17 is a view showing an internal configuration of the position adjustment robot 400. As shown, the lower sliding arm 420 and the upper sliding arm 430 are connected to the upper arm pivot axis 313a and the lower arm pivot axis 313b at the rear of the end effector 300 via the lower arm pivot axis connector 460 and the upper arm pivot axis connector 470, respectively.

The lower sliding arm 420 and upper sliding arm 430 have variable lengths exposed to the outside of the robot casing 410 by the lower arm driving unit 440 and the upper arm driving unit 450, respectively, and move the end effector 300 forward or backward in the X-axis direction or move the end effector upward or downward in the Y-axis direction.

The lower arm driving unit 440 and the upper arm driving unit 450 respectively include a lower arm driving motor 441 and an upper arm driving motor 451 configured to be forwardly or reversely driven by the navigation unit (not shown), a lower arm driving shaft 443 and an upper arm driving shaft 453 configured to be forwardly or reversely rotated by the lower arm driving motor 441 and the upper arm driving motor 451, a lower arm moving unit 445 configured to convert forward and reverse rotation of the lower arm driving shaft 443 into linear motion of the lower sliding arm 420 and an upper arm moving unit 455 configured to convert forward and reverse rotation of the upper arm driving shaft 453 into linear motion of the upper sliding arm 430, and a lower arm transmission means 447 and an upper arm transmission means 447 configured to transmit forward and reverse rotation of the lower arm driving motor 441 and the upper arm driving motor 451 to the lower arm driving shaft 443 and the upper arm driving shaft 453.

Each of the lower arm transmission means 447 and the upper arm transmission means 447 may be implemented in any of various forms, such as a belt or a gear. Although not shown in the figure, a thread is formed at an outer circumferential surface of each of the lower arm driving shaft 443 and the upper arm driving shaft 453, and the lower arm moving unit 445 and the upper arm moving unit 455 are threadedly coupled to the lower arm driving shaft 443 and the upper arm driving shaft 453, respectively. Accordingly, the lower arm moving unit 445 and the upper arm moving unit 455 move forward or backward along the lower arm driving shaft 443 and the upper arm driving shaft 453 depending on forward and reverse rotation of the lower arm driving shaft 443 and the upper arm driving shaft 453, respectively.

Forward movement of the lower arm moving unit 445 and the upper arm moving unit 455 increases the length of the lower sliding arm 420 and the upper sliding arm 430 exposed to the outside of the end effector 300.

FIG. 18 is an exploded perspective view showing a process in which the lower sliding arm 420 and the upper sliding arm 430 are coupled to the lower arm pivot axis 313b and the upper arm pivot axis 313a of the end effector 300 via the lower arm pivot axis connector 460 and the upper arm pivot axis connector 470, respectively.

As shown, a lower shaft insertion hole 461, into which the lower arm pivot axis 313b is inserted, is formed through the lower arm pivot axis connector 460. A lower arm coupling arm 463 is formed on one side of the lower arm pivot axis connector 460 so as to extend horizontally. The lower arm coupling arm 463 is disposed on one side of the lower sliding arm 420, a lower arm coupling connector 465 is disposed on the other side of the lower sliding arm, and a fastening member is inserted through the lower arm coupling arm 463, the lower sliding arm 420, and the lower arm coupling connector 465.

The lower arm pivot axis 313b is inserted into the lower pivot axis insertion hole 313-2 of the robot coupling cap 313 in a state of being inserted into the lower shaft insertion hole 461 and is connected to the lower sliding arm 420.

The upper arm pivot axis 313a is connected to the upper sliding arm 430 via the upper arm pivot axis connector 470 in the same manner.

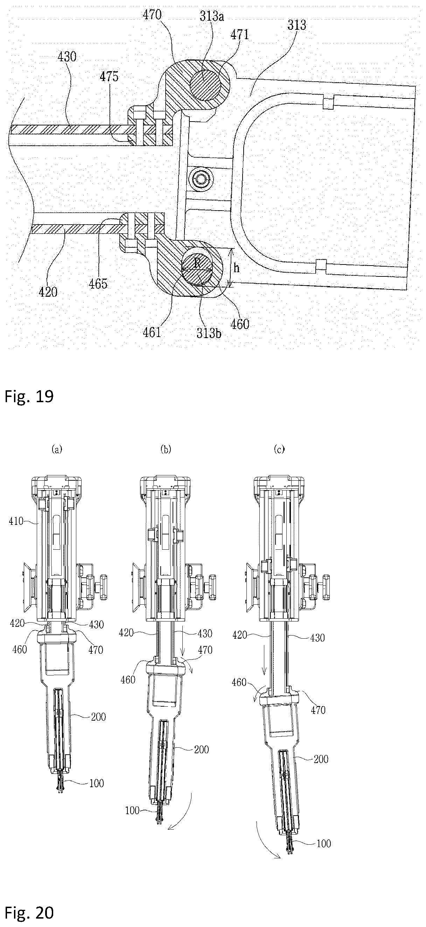

Here, as shown in FIG. 19, the lower shaft insertion hole 461 of the lower arm pivot axis connector 460 is formed in an oval shape having a height h greater than the diameter R of the lower arm pivot axis 313b, and the upper shaft insertion hole 471 of the upper arm pivot axis connector 470 is formed with a diameter equal to the diameter of the upper arm pivot axis 313a.

Accordingly, the casing unit 310 of the end effector 300 may be tilted upward and downward about the lower arm pivot axis 313b according to a change in the length of the lower sliding arm 420 and the upper sliding arm 430.

FIG. 20 is an illustrative view showing horizontal position adjustment and vertical position adjustment of the end effector 300 by the position adjustment robot 400.

As shown in (a) of FIG. 20, the lengths of the lower sliding arm 420 and the upper sliding arm 430 are shortened in an initial state in which the biopsy needle module 100 is coupled to the end effector 300. When the lower arm driving motor 441 and the upper arm driving motor 451 are rotated forward in this state, the lower sliding arm 420 and the upper sliding arm 430 coupled to the lower arm driving shaft 443 and the upper arm driving shaft 453 are moved forward by the same length.

When the lower sliding arm 420 and the upper sliding arm 430 are moved forward by the same length, the end effector 300 is moved forward only in the horizontal direction.

On the other hand, if the forward-movement length of the upper sliding arm 430 is greater than the forward-movement length of the lower sliding arm 420, as shown in (b) of FIG. 20, the end effector 300 is turned in the downward direction and the needle unit 110 of the biopsy needle module 100 is moved downward.

In addition, if the forward-movement length of the lower sliding arm 420 is greater than the forward-movement length of the upper sliding arm 430, as shown in (c) of FIG. 20, the end effector 300 is turned in the upward direction and the needle unit 110 of the biopsy needle module 100 is moved upward.

The X-axis position (horizontal direction) and the Y-axis position (vertical direction) of the needle unit 110 in the biopsy site M are adjusted by this mechanism. In addition, the Z-axis position of the needle unit is adjusted by the insertion depth of the needle unit 110 inserted into the biopsy site M in a bent state.

Consequently, the operator may accurately insert the needle unit 110 into the target biopsy location while viewing the captured image.

As described above, the biopsy needle manipulation robotic apparatus according to the present invention accurately guides the needle unit of the biopsy needle module coupled to the end effector to the target position of the biopsy site of the patient.

The biopsy needle manipulation robotic apparatus enables a precise biopsy procedure by accurately guiding the needle unit to the target position guided by the operator through the navigation unit in the horizontal direction, the height direction, and the depth direction.

In addition, the present invention has the advantage of being compatible with various types of medical imaging equipment by changing the height and the coupling width using the height adjustment fixing jig.

The embodiment of the biopsy needle manipulation robotic apparatus according to the present invention described above is exemplary only, and a person having ordinary skill in the art to which the present invention pertains will recognize that various modifications and other equivalent embodiments are possible therefrom. It will therefore be well understood that the present invention is not limited to the form recited in the above detailed description. Consequently, the true scope of technical protection of the present invention is to be determined by the technical ideas of the appended claims. In addition, the present invention is to be understood to include all variations, equivalents, and substitutes within the spirit and scope of the present invention as defined by the appended claims.

### [Description of Reference Symbols]

1: Biopsy system 10: Medical imaging equipment
11: Bed 13: Gantry
20: Fixing frame 30: Conventional biopsy needle device
31: Needle 40: End effector
100: Biopsy needle module 110: Needle unit
111: Outer needle 112: Outer needle fixing rib
113: Inner needle 113a: Tip
113b: Biopsy recess 114: Inner needle fixing rib
120: Needle bending frame 130: Needle guide
150: Locking member 151: Locking button
153: Button support arm 200: Biopsy needle manipulation robotic apparatus
300: End effector 310: Casing unit
311: Casing body 311a: Cap coupling wing
311b: Rear wall 313: Robot coupling cap
313-1: Upper pivot axis insertion hole 313-2: Lower pivot axis insertion hole
313a: Upper arm pivot axis 313b: Lower arm pivot axis
315: Cover 315a: Biopsy needle coupling recess
315b: Button coupling recess 317: Front cap
320: Trigger module 321: Module body
321-1: Inner needle rib insertion recess 321-2: Carrier movement path
321-3: Elastic shaft insertion hole 321-4: Module transfer shaft insertion hole
321-5: Outer needle driving shaft insertion hole 321-6: Screw tube coupling recess
323: Rear block 324: Front block
325: Outer needle carrier 325a: Outer needle rib insertion recess
325-2: Moving block 325-3: Elastic shaft insertion hole
325-4: Handle movement rail 325-5: Front hinge protrusion
325-6: Movement restriction stopper 326: Handle
326-1: Carrier pressing rib 326-2: Hinge pressing end
327: Elastic member support shaft 327a: Elastic member
328: Hinge 328-1: Body coupling end
328-3: Side protrusion 328-4: Rib insertion slope
330: Module transfer driving unit 331: Module transfer motor
333: Module transfer shaft 340: Outer needle driving unit
341: Outer needle driving motor 343: Outer needle driving shaft
345: Handle transfer screw tube 350: Shaft support plate
400: Position adjustment robot 410: Robot casing
411: Jig coupling protrusion 413: Upper arm exposure hole
415: Lower arm exposure hole 417: Robot front cover
419: Motor cap 420: Lower sliding arm
430: Upper sliding arm 440: Lower arm driving unit
441: Lower arm driving motor 443: Lower arm driving shaft
445: Lower arm moving nut 447: Lower arm transmission means
450: Upper arm driving unit 451: Upper arm driving motor
453: Upper arm driving shaft 455: Upper arm moving unit
457: Upper arm transmission means 460: Lower arm pivot axis connector
461: Lower shaft insertion hole 463: Lower arm coupling arm
465: Lower arm coupling connector 470: Upper arm pivot axis connector
471: Upper shaft insertion hole 473: Upper arm coupling arm
475: Upper arm coupling connector 500: Height adjustment fixing jig
510: Jig frame 511: Seating recess
513: Horizontal coupling position indication scale 520: Position adjustment frame
521: Coupling flange 521a: Coupling width adjustment slit
521b: Coupling width adjustment screw 523: Vertical height adjustment slit
523a: Vertical coupling height adjustment screw 524: Coupling height indication scale
530: Adapter 531: Protrusion fitting recess
540: Adapter fixing handle
A: Patient
M: Biopsy site
T: Target position
S: Tissue sample

## Claims

1. A biopsy needle manipulation robotic apparatus for manipulating a biopsy needle module such that a needle unit of the biopsy needle module is inserted into a biopsy site of a patient lying on a bed for medical imaging equipment to biopsy a tissue sample, wherein
the needle unit (110) of the biopsy needle module (100) comprises an outer needle (111) and an inner needle (113) overlapping each other, an inner needle fixing rib (114) fixedly coupled to a rear end of the inner needle (113), and an outer needle fixing rib (112) provided in front of the inner needle fixing rib (114), the outer needle fixing rib being fixedly coupled to a rear end of the outer needle (111), and
the biopsy needle manipulation robotic apparatus comprises:
an end effector (300) provided on a side surface of the bed (11), the end effector being configured to allow the inner needle fixing rib (114) and the outer needle fixing rib (112) to be coupled thereto by fitting, the end effector being configured to move each of the inner needle fixing rib (114) and the outer needle fixing rib (112) forward and backward such that the needle unit (110) penetrates the biopsy site of the patient to perform a biopsy procedure; and
a position adjustment robot (400) provided at a rear of the end effector (300) so as to have a predetermined length, the position adjustment robot being configured to adjust a horizontal position, a vertical position, and an insertion depth of the end effector (300) with respect to the bed such that the needle unit (110) penetrates a target position of the biopsy site.

2. The biopsy needle manipulation robotic apparatus according to claim 1, wherein the end effector (300) comprises:
a casing unit (310) disposed vertically on a side surface of the bed (11);
a trigger module (320) coupled to an interior of the casing unit (310) so as to be movable leftward and rightward, the trigger module being configured to allow the outer needle fixing rib (112) and the inner needle fixing rib (114) of the biopsy needle module (100) to be coupled thereto, the trigger module being configured to move the biopsy needle module (100);
a module transfer driving unit (330) comprising a module transfer shaft (333) extending through the trigger module (320) and horizontally provided at a lower part of the casing unit (310) and a module transfer motor (331) configured to forwardly and reversely rotate the module transfer shaft (333), the module transfer driving unit (330) being configured to forwardly or backwardly move the trigger module (320) toward or away from the biopsy site by forward and reverse rotation of the module transfer shaft (333) and to adjust a position of the inner needle (113); and
an outer needle driving shaft (343) extending through the trigger module (320) and horizontally disposed at an upper part of the module transfer shaft (333) so as to be coupled to the outer needle fixing rib (112) and an outer needle driving motor (341) configured to forwardly and reversely rotate the outer needle driving shaft (343).

3. The biopsy needle manipulation robotic apparatus according to claim 2, wherein
the trigger module (320) is provided at an upper part thereof with a carrier movement path (321-2) having a predetermined length,
the trigger module comprises:
an outer needle carrier (325) having an outer needle rib insertion recess (325a), into which the outer needle fixing rib (112) is inserted, formed on an upper surface thereof, the outer needle carrier being integrally provided at a lower part thereof with a moving block (325-2) coupled so as to be movable forward and backward along the carrier movement path (321-2), the moving block having a handle movement rail (325-4) recessed inward in both side surfaces of a lower part thereof;
a handle (326) coupled on a path of the outer needle driving shaft (343), the handle having a thread formed at an outer circumferential surface thereof, the handle being provided at one side thereof with a carrier pressing rib (326-1) configured to move along the handle movement rail (325-4) and to press the outer needle carrier (325) such that the outer needle carrier (325) is moved forward and backward, the a carrier pressing rib protruding from the one side of the handle;
a hinge (328) coupled to a lower part of the trigger module (320), the hinge being configured to restrict a front position and a rear position of the outer needle carrier (325) when the outer needle carrier (325) is moved forward and backward by the handle (326); and
an elastic member support shaft (327) extending and disposed through the trigger module (320) and an elastic member (327a) wound around an outer circumferential surface of the elastic member support shaft (327), the elastic member being compressed upon backward movement of the trigger module (320) to provide an elastic force to allow the trigger module (320) to be moved forward,
a front hinge protrusion (325-5) is formed on a front of the handle movement rail (325-4) so as to protrude outward therefrom, and a movement restriction stopper (325-6) is formed on a rear of the handle movement rail so as to protrude downward therefrom,
side protrusions (328-3) are formed on both sides of a rear end of the hinge (328) so as to protrude upward therefrom, and a rib insertion slope (328-4) is formed between the pair of side protrusions (328-3) so as to be inclined rightward and upward,
when the outer needle carrier (325) is backwardly moved by the handle (326), the front hinge protrusion (325-5) is moved along the side protrusions (328-3) and then caught by the side protrusions (328-3), whereby forward movement of the front hinge protrusion is restricted, and
a front end of the carrier pressing rib (326-1) of the handle (326) is formed in a shape corresponding to the rib insertion slope (328-4) such that the handle (326) is moved forward by driving of the outer needle driving shaft (343), the carrier pressing rib (326-1) is inserted into the rib insertion slope (328- 4), and the carrier pressing rib (326-1) is pressed to release a state in which the front hinge protrusion (325-5) is caught by the side protrusions (328-3).

4. The biopsy needle manipulation robotic apparatus according to claim 3, wherein
the casing unit is provided at a lower part and an upper part of a rear thereof with a lower arm pivot axis (313b) and an upper arm pivot axis (313a), respectively,
the position adjustment robot (400) comprises:
a robot casing (410) disposed vertically at a rear of the casing unit (310);
a lower arm driving unit (440) having a lower arm moving nut (455) disposed horizontally at a lower part of the robot casing (410);
an upper arm driving unit (450) having an upper arm moving nut (455) disposed horizontally on an upper part of the robotic casing (410);
a lower sliding arm (420) configured to horizontally connect the lower arm moving nut (455) to a rear end of the lower part of the casing unit (310);
an upper sliding arm (430) configured to horizontally connect the upper arm moving nut (455) to a rear end of an upper part of the casing unit (310); and
a lower arm pivot axis connector (460) and an upper arm pivot axis connector (470) configured to connect ends of the lower sliding arm (420) and the upper sliding arm (430) to the lower arm pivot axis (313b) and the upper arm pivot axis (313a), respectively,
a change in length of each of a lower arm driving shaft (443) and an upper arm driving shaft (453) is converted into a change in length of at least one of the lower sliding arm (420) and the upper sliding arm (430), and
the change in length of at least one of the lower sliding arm (420) and the upper sliding arm (430) is converted into upward and downward tilting of the casing unit (310) about the lower arm pivot axis (313b) and the upper arm pivot axis (313a).

5. The biopsy needle manipulation robotic apparatus according to claim 4, further comprising a position adjustment fixing jig 500 coupled to the bed (11) above a head of the patient, the position adjustment fixing jig being configured to adjut a horizontal position and a vertical height at which the position adjustment robot (400) is coupled to the bed (11).
